# EUROPEAN PATENT SPECIFICATION

(11) **EP 1 235 904 B1**
(45) Date of publication and mention of the grant of the patent: **12.01.2005**
(21) Application number: 00979206.0
(22) Date of filing: 20.11.2000
(51) Int. Cl.: C12N 15/00

(54) **FRUCTOSE POLYMER SYNTHESIS IN MONOCOT PLASTIDS**
FRUKTOSEPOLYMER SYNTHESE IN PLASTIDEN MONOKOTYLER PLANZEN
SYNTHESE DE FRUCTOSE POLYMERE DANS DES PLASTES MONOCOTYLEDONES

(30) Priority: 18.11.1999 US 166268 P
(43) Date of publication of application: 04.09.2002
(60) Divisional application: 04026319.6
(73) Proprietor: E.I. DU PONT DE NEMOURS AND COMPANY, Wilmington, Delaware 19898 (US)
(72) Inventor: CAIMI, Perry, G., Kennett Square, PA 19348 (US); LIGHTNER, Jonathan, Mulino, OR 97042 (US)
(74) Representative: Webber, Philip Michael
(86) International application number: PCT/US2000/031788
(87) International publication number: WO 2001/036622

(56) References cited:
- WO-A-99/46395
- CAIMI P G ET AL: "Cytosolic expression of the Bacillus amyloliquefaciens SacB protein inhibits tissue development in transgenic tobacco and potato." NEW PHYTOLOGIST, vol. 136, no. 1, 1997, pages 19-28, XP002166948 ISSN: 0028-646X
- CAIMI PERRY G ET AL: "Fructan accumulation and sucrose metabolism in transgenic maize endosperm expressing a Bacillus amyloliquefaciens SacB gene." PLANT PHYSIOLOGY (ROCKVILLE), vol. 110, no. 2, 1996, pages 355-363, XP000999065 ISSN: 0032-0889

## Description

### FIELD OF THE INVENTION

The present invention concerns methods for synthesis and accumulation of fructo-oligosaccharides (FOS) in monocots. The invention also describes targeting fructosyltransferase enzymes (FTFs) to the plastid, resulting in a mixture of starch and FOS which exhibit functional properties distinctly different compared to wild-type (dent) starch.

### BACKGROUND OF THE INVENTION

Higher plants accumulate commercially useful carbohydrate polymers such as cellulose, starch and FOS. Starch and cellulose may be useful in their native form, but are much more likely to be enzymatically or chemically modified, greatly increasing their value.

FOS is a linear or branched polymer of repeating fructose residues. The number of residues within an individual polymer varies, depending on the source. *(Science and Technology of Fructans*, (1993) M. Suzuki and N. Chatterton, eds. CRC Press Inc., Boca Raton, FL pp. 169-190). FOS is an ideal additive for use in a wide range of no-calorie food products because humans cannot metabolize FOS and polymers with three to four fructose residues (DP 3-4) are relatively sweet. High DP polymers (>DP 4) are not sweet, however, they can provide texture to food products very similar to that of fat. High DP fructo-oligosaccharide used as a fat replacer contributes little to the caloric value of the product. Fructo-oligosaccharide is also considered to be an excellent source of fructose for the production of high fructose syrup (Fuchs, A. (1993) in *Science and Technology of Fructans*, M. Suzuki and N. Chatterton, eds. CRC Press Inc., Boca Raton, FL pp. 319-352). Simple hydrolysis of FOS into individual fructose residues would have a tremendous advantage over the current, technically demanding process of enzymatically converting starch into high fructose syrup. Thus using FOS as the starting material could significantly reduce fructose production costs.

The commercial value for FOS is potentially high, however, its use is severely limited due to its high cost of production. Fructo-oligosaccharides for use in high-value, low-calorie foods are normally produced by expensive fermentation culture technology. Isolation from plants would reduce production costs, however, FOS is not found in many crops of agricultural importance. Traditional crops that are adapted to wide growing regions, such as oat, wheat and barley accumulate FOS, but only at extremely low levels. Fructo-oligosaccharide is currently harvested from plants on a relatively small commercial scale and only from a single plant species, *Cichorium intybus.*

Transgenic versions of major crops that accumulate FOS through expression of chimeric FTF genes would have a significant advantage over native FOS-storing plants by making use of established breeding programs, pest resistance and adaptation to a wide variety of growing regions throughout the world. Examples of FOS synthesis in transgenic plants containing genes from bacterial species, such as *Bacillus, Streptococcus* and *Erwinia* have been reported (Caimi et al., (1996) *Plant Physiol*. *110*:355-363; Ebskamp et al., (1994) *Biotechnol*. *12*:272-275; Rober et al., (1996) *Planta 199*:528-536).

Directing polymer synthesis to specific cellular compartments has been shown to be critical to normal plant development (Caimi et al., (1996) *Plant Physiol*. *110*:355-363). Targeting a chimeric FTF to the cytosol can be extremely detrimental to cell development whereas, synthesis of FOS in the cell vacuole has been shown to be less detrimental to tissue, however, very low levels of FOS accumulation were reported (Ebskamp et al., (1994) *Biotechnol*. *12*:272-275). Engineering increased levels of FOS in transgenic plants, by targeting FOS synthesis to specific cellular compartments would minimally affect cell development and allow increased efficiency of isolation and purification of product. This, in turn, would result in reduced cost of production.

Directing FOS synthesis to plastids could also provide an opportunity to blend FOS and starch synthesis to produce starch blends. Starch is a mixture of two polysaccharides, amylose and amylopectin. Amylose is an unbranched chain of up to several thousand α-D-glucopyranose units linked by α-1,4 glycosidic bonds. Amylopectin is a highly branched molecule made up of up to 50,000 α-D-glucopyranose residues linked by α-1,4 and α-1,6 glycosidic bonds. Approximately 5% of the glycosidic linkages in amylopectin are α-1,6 bonds, which leads to the branched structure of the polymer.

Functional properties, such as viscosity and stability of a gelatinized starch determine the usefulness and hence the value of starch in food and industrial applications. Where a specific functional property is needed, starches obtained from various crops such as corn, rice, or potatoes may meet the functionality requirements. If a starch does not meet a required functional property the functionality can sometimes be achieved by chemically modifying the starch. Various types and degrees of chemical modification are used in the starch industry and the use of chemically modified starches in the United States is regulated by the Food and Drug Administration (FDA). "Food starch-modified" starches may be used in food but must meet specified treatment limits, and " industrial starch-modified" starches may be used in items such as containers that come in contact with food and must also meet specified treatment requirements; Code of Federal Regulations, Title 21, Chapter 1, Part 172, Food Additives Permitted in Food for Human Consumption, Section 172, 892, Food Starch-Modified, U. S. Government Printing Office, Washington, DC 1981; (a) Part 178, Indirect Food Additives, Sect. 178.3520, Industrial Starch-Modified. These regulations limit the degree of chemical modification by defining the maximum amount of chemical reagent that can be used in the modification steps. The levels of by-products in starch resulting from the modification process are also regulated. For example, propylene chlorohydrin residues in hydroxypropyl starch are of special concern; Tuschhoff, J. V., (1986) Hydroxypropylated Starches, In Modified Starches: Properties and Uses, O. B. Wurzburg, ed., CRC Press, Boca Raton, FL, pp. 55-57. Creating functionality, through means other than with chemical modification would eliminate concerns relating to chemical "residues" remaining in these regulated starch products.

The physical properties of unmodified starch during heating and cooling limit its usefulness in many applications. As a result, considerable effort and cost is needed to chemically modify starch in order to overcome these limitations and to expand the usefulness of starch in industrial applications. Some limitations of unmodified starches and properties of modified starches are listed in *Modified Starches: Properties and Uses,* O. B. Wurzburg, ed., (1986) CRC Press Inc., Boca Raton, FL.

Chemical modifications are often used to stabilize starch granules thereby making the starch suitable for thousands of food and industrial applications including baby foods, powdered coffee creamer, surgical dusting powders, paper and yarn sizings and adhesives. Common chemical modifications include cross linking in which chemical bonds are introduced to act as stabilizing bridges between starch molecules, and substitution in which substituent groups such as hydroxyethyl, hydroxypropyl or acetyl groups are introduced into starch molecules.

Substituent groups on starch molecules interfere with inter and intra-chain attraction between hydroxyl groups. Methods that alter chain association significantly affect the properties of starch (Orthoefer, F. T. (1987) in *Corn: Chemistry and Technology,* S. A. Watson and P. E. Ramstad, eds, American Association of Cereal Chemists, Inc, St. Paul, MN pp. 479-499). One example, hydroxypropylated starches, are widely used in the food industry due to their improved viscosity stability and water-holding capacity under low-temperature storage conditions (Rutenberg, M. W. and Solarek, D., (1984) Starch Derivatives, in *Starch* R. L. Whistler, J. N. BeMiller, and E. F. Paschall eds., Academic Press, Inc. Orlando FL, pp 312-366).

Targeting an FTF to plastids, results in a starch/FOS mixture, which may alter starch structure and functionality. Targeting an FTF to plastids has been reported (Turk et al. WO 97/29186). However, Turk et al. (WO 97/29186) fails to report FOS concentration within transgenic plants. Expression only in transgenic dicotyledenous (dicots) plants is described and specific modification of starch functional properties is not demonstrated.

The present invention describes, *inter alia*, a method of synthesizing high levels of FOS in transgenic monocotyledonous plants containing an endosperm-specific promoter directing expression of an plastid-targeted FTF gene. Targeting this enzyme to plastids in maize endosperm, for example, was shown to alter the physical and functional properties of maize starch. This is the first example of altered starch functionality within a monocotyledonous (monocot) plant by accumulating an alternative polymer within the granule.

Numerous differences between monocots and dicotyledonous (dicots) plants are known which render the practical application of techniques, manipulations or methods for one group of plants inapplicable to the other. This is especially true when considering the differences in carbohydrate profile within storage tissue among dissimilar plants. Well-documented differences between starches isolated from mono- and dicots have been exploited throughout history. The functional difference between potato and maize starches, or between rice and tapioca starches are but two examples. These significant differences are well recognized by the starch industry as evidenced by the existence of markets for starches from a variety of plant species.

Starch synthesis in storage tissues from cereals and non-cereals depends upon different types of metabolic precursors. In contrast to starch synthesis in cereal amyloplasts, storage plastids from many plants import hexose phosphates (Mohlmann et al., (1997) *Biochem J*. *324*:503-509). For example, pea *(Pisum sativum* L.) a dicot, transports glucose-6-phosphate into amyloplasts, the site where starch is synthesized and stored. In monocots, such as maize, ADPglucose is transported into the amyloplast. (Denyer et al., (1996) *Plant Phys*. *112*:779-785). This illustrates the profound difference in metabolic pathways necessary for processing various forms of carbohydrate transported into plastids.

Based on these reports, it is clear that there are significant differences in the carbohydrate content of monocots and dicots. Sucrose is the sole substrate, utilized by FTFs in the synthesis of FOS. The presence of sucrose within the plastids of monocot cells (e.g. maize endosperm cells) has not been reported to Applicant's knowledge. The concentration of sucrose is also critical to FOS synthesis and again, there are no reports known to Applicant of sucrose concentration in monocot plastids. Therefore, prior to the instant invention, predicting whether substrate is available for synthesis of FOS in monocot plastids was not possible. Furthermore, prior to the instant invention, targeting an FTF to plastids in various plant species would have produced unexpected results with regard to FOS synthesis.

While not intending to be bound by any particular theory or theories of operation, it is believed that a critical component in FOS synthesis is the availability of sucrose within a transgenic plant. Sucrose transport and metabolism among plant species differs considerably. Specialized cells (basal endosperm transfer cells or BET cells) are adapted for the transport and metabolism of sucrose in developing maize, wheat and barley kernels (Olsen et al. (1999) *Trend in Plant Sci*. *4*:253-257). The majority (greater than 90%) of sucrose transported to maize seeds is thought to be hydrolyzed in the BET layer (Shannon, J. (1972) *Plant Physiol*. *49*:198-202). The resulting hexose sugars are transported to the developing endosperm cells and re-synthesized as sucrose prior to entering the starch biosynthetic pathway. These specialized transfer cells are not known in dicot species. In contrast to maize, sucrose is directly transported to potato (a dicot) tuber cells where it enters the starch biosynthetic pathway in an unmodified form. (Oparka, K. and Wright, K. (1988) *Planta 174*:123-126). Variations in sucrose concentration, therefore, are to be expected in storage tissue, depending on the plant species.

Starch synthesis in storage tissues from cereals and non-cereals depends upon different types of metabolic precursors. In contrast to starch synthesis in cereal amyloplasts, storage plastids from many plants import hexose phosphates (Mohlmann et al., (1997) *Biochem J*. *324*:503-509). For example, pea *(Pisum sativum* L.) a dicot, transports glucose-6-phosphate into amyloplasts, the site where starch is synthesized and stored. In monocots, such as maize, ADPglucose is transported into the amyloplast. (Denyer et al., (1996) *Plant Phys*. *112*:779-785). This illustrates the profound difference in metabolic pathways necessary for processing various forms of carbohydrate transported into plastids.

Based on these reports, it is clear that there are significant differences in the carbohydrate content of monocots and dicots. Sucrose is the sole substrate, utilized by FTFs in the synthesis of FOS. The presence of sucrose within the plastid of maize endosperm cell has not been reported to Applicant's knowledge. The concentration of sucrose is also critical to FOS synthesis and again, there are no reports known to Applicant of sucrose concentration in maize endosperm plastids. Therefore, prior to the instant invention, predicting whether substrate is available for synthesis of FOS in maize plastids was not possible. Furthermore, prior to the instant invention, targeting an FTF to plastids in various plant species would have produced unexpected results with regard to FOS synthesis.

While not intending to be bound by any particular theory or theories of operation, it is believed that a critical component in FOS synthesis is the availability of sucrose within a transgenic plant. Sucrose transport and metabolism among plant species differs considerably. Specialized cells (basal endosperm transfer cells or BET cells) are adapted for the transport and metabolism of sucrose in maize kernels. The majority (greater than 90%) of sucrose transported to maize seeds is thought to be hydrolyzed in the BET layer (Shannon, J. (1972) *Plant Physiol*. *49*:198-202). The resulting hexose sugars are transported to the developing endosperm cells and re-synthesized as sucrose prior to entering the starch biosynthetic pathway. These specialized transfer cells have not been found in dicot species. In contrast to maize, sucrose is directly transported to potato (a dicot) tuber cells where it enters the starch biosynthetic pathway in an unmodified form. (Oparka, K. and Wright, K. (1988) *Planta 174*:123-126). Variations in sucrose concentration, therefore, are to be expected in storage tissue, depending on the plant species.

Although sometimes poorly understood, exploiting the differences between monocot and dicot plants should not be considered a new concept. These differences are the driving force in commercialization of herbicides. One example is the herbicide 2-4-D, which is tremendously toxic to dicots, but has little or no effect on monocot species.

Variations in carbohydrate concentration, transport and metabolism among plant species, especially between monocots and dicots, are clearly too consequential to allow indiscriminate generalizations. These few, but significant examples clearly point to the futile nature of predictions regarding the success of FOS synthesis or altering starch functionality in the plastid of a monocot based on various reports of investigations in dicots.

### SUMMARY OF THE INVENTION

The present invention discloses a method for producing increased levels of fructose polymers and a starch/fructo-oligosaccharide blend, through expression of a plastid-targeted fructosyltransferase gene sequence in transgenic monocot species. The invention is also directed to a recombinant DNA construct comprising a tissue specific promoter, operably linked to a plastid targeting signal, operably linked to the coding sequence for a fructosyltransferase gene (for example, EC 2.4.1.10) such that said construct is capable of transforming a monocot cell resulting in production of fructo-oligosaccharide within a plastid. The invention further describes the altered physical and functional properties of a mixture of starch and fructo-oligosaccharide within the granule isolated from said corn plant cell. The invention further concerns a method of producing a fructo-oligosaccharide/starch mixture comprising growing a transgenic monocot plant (harboring a plastid-targeted fructosyltransferase gene), harvesting said plant, and extracting the fructo-oligosaccharide/starch mixture from the harvested plant. The starch and fructo-oligosaccharide mixture or blend refers to the co-mingling of starch and fructo-oligosaccharide. The polymers may be linked covalently or incidentally. The polymers may be surrounding one another or completely entangled and that association alters functional properties compared to starch isolated from non-transformed plants.

The present invention is directed to a construct comprising an isolated and/or purified FTF gene, as well as altered versions thereof. Alterations may influence the activity of the fructosyltransferase in such a way that, for example, the degree of polymerization or the structure of the fructo-oligosaccharide produced is altered. Furthermore, according to the present invention a single fructosyltransferase gene sequence or a combination of fructosyltransferase gene sequences may be used.

The induced accumulation of fructo-oligosaccharide in transgenic plants as described by the present invention will allow for the extraction of fructo-oligosaccharide from these plants for the purpose of fructo-oligosaccharide production. Fructo-oligosaccharide can accumulate in these plants in harvestable organs such as roots, leaves, stems and seeds. Furthermore, the present invention relates to seeds, cuttings or other parts of the transgenic plants which are useful for the continuous production of further generations of said plants.

The fructo-oligosaccharides produced using transgenic plants of the present invention may be used in various food and non-food applications. Examples include, and are not limited to, human and animal food products, the production of fructose syrups and the production of chemicals and plastics either as such or in modified form.

Genetically modified crop plants, which incorporate the fructosyltransferase-encoding constructs mentioned above allow for the efficient production of high quality carbohydrate polymers.

### BRIEF DESCRIPTION OF THE DRAWINGS AND SEQUENCE DESCRIPTIONS

The invention can be more fully understood from the following detailed description and the accompanying drawings and sequence descriptions, which form a part of this application.
Figure 1 is a schematic representation of the construct (10kD-CTS-Sac) used to express the plastid-targeted SacB gene in maize endosperm. The chloroplast transit signal was fused, in frame, to the mature coding sequence of the SacB gene.
Figure 2 is a graphic illustration of the concentration of FOS isolated from ten independent transformed maize lines containing the 10kD-CTS-Sac construct. Ten mature seeds were assayed separately for FOS concentration then averaged for each independent transgenic line. The graph represents the average concentration of FOS in mg per seed.
Figure 3 is a TLC plate showing the presence of FOS in two separate transgenic lines. The EtOH extract (3 ul) has been spotted on a TLC plate, in addition to the water extract of the pellet (3 ul). The positive signal at the origin of the plate indicates that FOS accumulated in the seeds is high molecular weight. Soluble sugars (sucrose and fructose) are present near the solvent front in the EtOH extracts in addition to a small amount of FOS.
Figure 4 is a scanning electron micrograph of starch granules isolated from seeds containing the 10kD-CTS-Sac construct.
Figure 5 is a comparison of the molecular weight distribution of starch from seeds with or without the 10kD-CTS-Sac construct. An increase in high molecular weight carbohydrate is demonstrated by the sample XBG02118-5.
Figure 6 shows water extracts (3 ul) of isolated starch granules spotted on a TLC plate. Granules were isolated from individual seeds segregating for the 10kD-CTS-Sac construct. Positive signal is present at the origin of the plate in the samples S1, S2 and S3 from two independent lines (XBG02045 and XBG02118).
Figure 7 shows RVA analysis from dent and transgenic lines containing the 10kD-CTS-Sac construct (XAW00361 and XAW00363). Peak viscosity, pasting temperature and onset of pasting are all altered in the transgenic lines, compared to native dent starch.

SEQ ID NO:1 is a partial nucleotide sequence representing the maize (*Zea mays* L.) small subunit RuBP-carboxylase chloroplast transit signal.
SEQ ID NO:2 is a partial amino acid sequence representing the maize (*Zea mays* L.) small subunit RuBP-carboxylase chloroplast transit signal encoded by SEQ ID NO:1.

### DETAILED DESCRIPTION OF THE INVENTION DEFINITIONS

In the context of this disclosure, a number of terms shall be utilized. As used herein, the term "nucleic acid" refers to a polymer of RNA or DNA that is single- or double-stranded, optionally containing synthetic, non-natural or altered nucleotide bases. A "nucleic acid fragment" is a fraction of a given nucleic acid molecule. In higher plants, deoxyribonucleic acid (DNA) is the genetic material while ribonucleic acid (RNA) is involved in the transfer of the information in DNA into proteins. A nucleic acid fragment in the form of a polymer of DNA may be comprised of one or more segments of cDNA, genomic DNA, synthetic DNA or mixtures thereof.

A "genome" is the entire body of genetic material contained in each cell of an organism. The term "nucleotide sequence" refers to a polymer of DNA or RNA which can be single- or double-stranded, optionally containing synthetic, non-natural or altered nucleotide bases capable of incorporation into DNA or RNA polymers.

"Gene" refers to a nucleic acid fragment that expresses a specific protein, including regulatory sequences preceding (5' non-coding sequences) and following (3' non-coding sequences) the coding sequence. "Native gene" refers to a gene as found in nature with its own regulatory sequences. "Chimeric gene" refers any gene that is not a native gene, comprising regulatory and coding sequences that are not found together in nature. Accordingly, a chimeric gene may comprise regulatory sequences and coding sequences that are derived from different sources, or regulatory sequences and coding sequences derived from the same source, but arranged in a manner different than that found in nature. "Endogenous gene" refers to a native gene in its natural location in the genome of an organism. A "foreign" gene refers to a gene not normally found in the host organism, but that is introduced into the host organism by gene transfer. Foreign genes can comprise native genes inserted into a non-native organism, or chimeric genes. A "transgene" is a gene that has been introduced into the genome by a transformation procedure.

"Coding sequence" refers to a nucleotide sequence that codes for a specific amino acid sequence, or at least a portion thereof that provides an amino acid sequence that functions substantially the same as a full length amino acid sequence. "Regulatory sequences" refer to nucleotide sequences located upstream (5' non-coding sequences), within, or downstream (3' non-coding sequences) of a coding sequence, and which influence the transcription, RNA processing or stability, or translation of the associated coding sequence. Regulatory sequences may include promoters, translation leader sequences, introns, and polyadenylation recognition sequences.

"Initiation codon" and "termination codon" refer to a unit of three adjacent nucleotides in a coding sequence that specifies initiation and chain termination, respectively, of protein synthesis (mRNA translation). "Open reading frame" refers to the amino acid sequence encoded between translation initiation and termination codons of a coding sequence.

As used herein, suitable "regulatory sequences" refer to nucleotide sequences located upstream (5'), within, and/or downstream (3') to a coding sequence, which control the transcription and/or expression of the coding sequences. These regulatory sequences include promoters, translation leader sequences, transcription termination sequences, and polyadenylation sequences. In artificial DNA constructs, regulatory sequences can also control the transcription and stability of antisense RNA.

"Promoter" refers to a nucleotide sequence capable of controlling the expression of a coding sequence or functional RNA. In general, a coding sequence is located 3' to a promoter sequence. The promoter sequence consists of proximal and more distal upstream elements, the latter elements often referred to as enhancers. Accordingly, an "enhancer" is a nucleotide sequence which can stimulate promoter activity and may be an innate element of the promoter or a heterologous element inserted to enhance the level or tissue-specificity of a promoter. Promoters may be derived in their entirety from a native gene, or be composed of different elements derived from different promoters found in nature, or even comprise synthetic nucleotide segments. It is understood by those skilled in the art that different promoters may direct the expression of a gene in different tissues or cell types, or at different stages of development, or in response to different environmental conditions. Promoters which cause a nucleic acid fragment to be expressed in most cell types at most times are commonly referred to as "constitutive promoters". "Tissue-specific" or "development-specific" promoters as referred to herein are those that direct gene expression almost exclusively in specific tissues, such as leaves or seeds, or at specific developmental stages in a tissue, such as in early or late embryogenesis, respectively. New promoters of various types useful in plant cells are constantly being discovered; numerous examples may be found in the compilation by Okamuro and Goldberg, (1989) *Biochemistry of Plants 15*:1-82. It is further recognized that since in most cases the exact boundaries of regulatory sequences have not been completely defined, nucleic acid fragments of different lengths may have identical promoter activity.

"RNA transcript" refers to the product resulting from RNA polymerase-catalyzed transcription of a DNA sequence. When the RNA transcript is a perfect complementary copy of the DNA sequence, it is referred to as the primary transcript or it may be a RNA sequence derived from posttranscriptional processing of the primary transcript and is referred to as the mature RNA. "Messenger RNA (mRNA)" refers to the RNA that is without introns and that can be translated into polypeptide by the cell. "cDNA" refers to a double-stranded DNA that is complementary to and derived from mRNA. "Sense" RNA refers to an RNA transcript that includes the mRNA and so can be translated into a polypeptide by the cell. "Antisense RNA" refers to an RNA transcript that is complementary to all or part of a target primary transcript or mRNA and that blocks the expression of a target gene (see U.S. Patent No. 5,107,065). The complementarity of an antisense RNA may be with any part of the specific nucleotide sequence, i.e., at the 5' non-coding sequence, 3' non-coding sequence, introns, or the coding sequence. "Functional RNA" refers to sense RNA, antisense RNA, ribozyme RNA, or other RNA that may not be translated but yet has an effect on cellular processes.

The term "operably linked" refers to the association of two or more nucleic acid fragments on a single nucleic acid fragment so that the function of one is affected by the other. For example, a promoter is operably linked with a coding sequence when it is capable of affecting the expression of that coding sequence (i.e., that the coding sequence is under the transcriptional control of the promoter). Coding sequences can be operably linked to regulatory sequences in sense or antisense orientation.

The term "expression", as used herein, refers to the transcription and stable accumulation of sense (mRNA) or antisense RNA derived from the nucleic acid fragment of the invention. Expression may also refer to translation of mRNA into a polypeptide. "Antisense inhibition" refers to the production of antisense RNA transcripts capable of suppressing the expression of the target protein. "Overexpression" refers to the production of a gene product in transgenic organisms that exceeds levels of production in normal or non-transformed organisms. "Co-suppression" refers to the production of sense RNA transcripts capable of suppressing the expression of identical or substantially similar foreign or endogenous genes (U.S. Patent No. 5,231,020).

"Altered levels" refers to the production of gene product(s) in transgenic organisms in amounts or proportions that differ from that of normal or non-transformed organisms. "Altered" rheological properties of the starch-fructo-oligosaccharide blend refers to an increase or decrease in a rheological property relative to that property in a solution of starch, wherein the solution of starch is substantially lacking in fructo-oligosaccharides and provided that the starch solution comprises substantially the same amount of starch as the blend. Preferably, such starch solution substantially lacking in fructo-oligosaccharides is a fructo-oligosaccharide-free starch solution. That is the solution contains no fructo-oligosaccharides, however, a minimal amount of fructo-oligosaccharides may be present, as a result of contamination for example. Thus, in accordance with the present invention, blends comprising any percentage of starch are compared relative to a starch solution having no fructo-oligosaccharides comprising substantially the same amount of starch as the blend.

"Increase" for purposes of the present invention includes and is not limited to raise, to make greater, augment, amplify, enhance and heighten. "Decrease" for purposes of the present invention includes and is not limited to reduce, decline, lessen, inhibit and prevent.

Rheological properties capable of being altered in the starch-fructo-oligosaccharide blend include pasting temperature and final viscosity. Pasting temperature (also known as gelatinization temperature of starch) refers to the onset temperature of the rise in viscosity of a starch solution as the solution is heated. H. F. Zobel (1984) in *Starch: Chemistry and Technology,* R. L.Whistler, J. N. BeMiller and E. F. Paschall, eds., Academic Press Inc., San Diego, CA, pp. 285-309. The pasting temperature of the starch-fructo-oligosaccharide blend may be increased or decreased. An increase in pasting temperature of about 30% to about 1%, more preferably about 2%, 3%, 4%, 5%, 6%, 7%, 8%, 9%, 10%, 11%, 12%, 13%, 14%, 15%, 16%, 17%, 18%, 19%, 20%, 21 %, 22%, 23%, 24%, 25%, 26%, 27%, 28% or 29%, of the starch-fructo-oligosaccharide blend relative to the pasting temperature of a starch solution containing no fructo-oligosaccharides is observed. Preferably, a decrease in pasting temperature of about 30% to about 1%, more preferably about 27% to about 22%, of the starch-fructo-oligosaccharide blend of the present invention is observed over the pasting temperature of the starch solution having no fructo-oligosaccharides.

Final viscosity refers to the viscosity of a solution at the conclusion of a cooking experiment commonly referred to as rapid visco analysis, in which the solution is heated, held at a stable temperature and then cooled and held stable again, while the viscosity is measured. The term "final viscosity" and such experiments are well known to skilled artisans and disclosed for example in F. T. Orthoefer (1987) in *Corn: Chemistry and Technology,* S. A. Watson and P. E. Ramstad, eds. American Association of Cereal Chemists, St. Paul, MN, pp. 479-499. The final viscosity of the starch-fructo-oligosaccharide blend may be increased or decreased. Preferably, an increase in final viscosity such that the final viscosity is 600% (6-fold) to about 20%, more preferably about 500% to about 30%, of a starch solution having no fructo-oligosaccharides. A decrease in final viscosity such that the final viscosity is about 10% to about 30%, more preferably about 20%, of a starch solution containing no fructo-oligosaccharides.

"Mature" protein refers to a post-translationally processed polypeptide; i.e., one from which any pre- or propeptides present in the primary translation product have been removed. "Precursor" protein refers to the primary product of translation of mRNA; i.e., with pre- and propeptides still present. Pre- and propeptides may be, and are not limited to, intracellular localization signals.

The "3' non-coding sequences" refer to nucleotide sequences located downstream of a coding sequence and include polyadenylation recognition sequences and other sequences encoding regulatory signals capable of affecting mRNA processing or gene expression. The polyadenylation signal is usually characterized by affecting the addition of polyadenylic acid tracts to the 3' end of the mRNA precursor. The use of different 3' non-coding sequences is exemplified by Ingelbrecht et al., (1989) *Plant Cell 1*:671-680.

A "chloroplast-targeting signal" or "plastid-targeting sequence" is an amino acid sequence which is translated in conjunction with a protein and directs the protein to the chloroplast or other plastid types, such as an amyloplasts, present in the cell. "Chloroplast- or plastid-targeting signal sequence" refers to a nucleotide sequence that encodes a chloroplast- or plastid-targeting signal peptide. Amyloplasts refer to organelles in which reserve starch is synthesized in higher plant species.

A fructosyltransferase enzyme for use in the construct of the present invention may be provided by plants such as and not limited to monocots *(Triticum aestivum* L., for example) and dicots *(Cicorium intybus* L., for example) and bacterial sources such as and not limited to *Bacilllus amyloliquefaciens*, *Streptococcus mutans* and *Erwinia herbicola* as well as fungal sources (*Aspergillus niger* and *Aspergillus sydowi,* for example).

"Transformation" refers to the transfer of a nucleic acid fragment into the genome of a host organism, resulting in genetically stable inheritance. Host organisms containing the transformed nucleic acid fragments are referred to as "transgenic" organisms. Examples of methods of plant transformation include *Agrobacterium-mediated* transformation (De Blaere et al. (1987) *Meth. Enzymol*. *143*:277) and particle-accelerated or "gene gun" transformation technology (Klein et al. (1987) *Nature (London) 327*:70-73; U.S. Patent No. 4,945,050).

Standard recombinant DNA and molecular cloning techniques used herein are well known in the art and are described more fully in Sambrook, J., Fritsch, E.F. and Maniatis, T. *Molecular Cloning: A Laboratory Manual*; Cold Spring Harbor Laboratory Press: Cold Spring Harbor, 1989 (hereinafter "Maniatis").

"Fructosyltransferase" refers to a protein coded for by any one of several plant or microbial genes having the property of producing a carbohydrate polymer consisting of repeating fructose residues. The repeating fructose residues may be linked β 2-1 linkage or a β 2-6 linkage or any combination of the two linkage types. The polymer of repeating fructose residues may contain one terminal glucose residue, derived from a sucrose molecule, and at least two fructose residues. The polymer of repeating fructose residues in any form, with any combination of linkages, and with any number of fructose residues, is referred to generally as fructyl-oligosaccharides or "FOS".

A "fructosyltransferase gene" refers to a DNA sequence that includes at least a part of a coding sequence for a fructosyltransferase protein. "Plastid" refers to a membrane bound organelle in plant cells, which is the site of carbohydrate manufacture or storage. The term "plastid-targeted" refers to a protein containing signal sequences, which result in the delivery of the protein to and within the plastid. "Functionality" or "functional properties" refer to the specific rheological properties of a polymer. The rheological properties impart a specific characteristic on the polymer (usually, but not limited to starch) such that the polymers become useful or more useful in a specific food/non-food application.

The term "FOS/starch" or "starch/FOS" mixture or blend refers to the co-mingling of starch and FOS. The polymers may be linked covalently or incidentally. The polymers may be surrounding one another or completely entangled. The actual association of FOS and starch within the granule is not critical, except that association alters physical or functional properties compared to starch isolated from non-transformed plants. The starch-fruto-oligosaccharide blend preferably comprises a ratio (by weight) of starch to fructo-oligosaccharides from about 10:1 to about 1:10, more preferably about 7:1 to about 1:7.

The present invention describes chimeric constructs comprising tissue specific regulatory sequences and an FTF coding sequence. The chimeric construct is capable of synthesizing a fructose polymer using sucrose as a substrate when expressed in a transgenic monocot plant. Expression of the FTF gene results in the synthesis of fructose polymers, useful in numerous food and industrial applications. A transgenic corn plant (*Zea mays* L.) properly expressing the FTF gene, distinguishes itself from a generic plant of the same species by the presence of FOS accumulation in the mature seeds.

The present invention is directed to plants, more particularly, monocots, i.e., plants producing reserve starch in triploid endosperm tissue, more particularly plants from the family *Poaceae,* including and not limited to corn, wheat, barley, rye, triticale, oats, and sorghum.

Transfer of the nucleic acid fragments of this invention into a plant directs expression of an FTF protein in a manner that results in accumulation of fructose polymers, without concern for loss or alteration of the polymer due to plant degratory enzymes during harvest, transport, or storage and without the loss of established co-products from any particular species. Transgenic crops containing chimeric genes comprising tissue specific regulatory sequences and an FTF gene sequence will provide a renewable source of fructose polymers. Accumulation of FOS will be determined in part, by the level of expression of the chimeric gene in transformed crops. The level of expression depends in part, on the tissue specific expression signals, the number of copies of the gene integrated into the plant genome and location of gene integration; FOS accumulation may also be subject to substrate availability. The amount of substrate available to the enzyme may be determined by the species (including mutants within a species), the tissue type where expression occurs, the subcellular location of expression and the stage of development of a particular plant. The stability of the introduced protein may also influence FOS accumulation and depends in part, on its proper processing, intracellular targeting and ability to function in a foreign environment.

Successful expression of a gene with carbohydrate metabolic properties such as the *Bacillus amyloliquefaciens* SacB gene, in a transgenic plant would require consideration of the following factors: (1) the species transformed, (2) the specific tissue where expression is to occur, (3) the specific cellular compartment where the enzyme is to be targeted (4) and the timing of expression. All of these factors must be carefully coordinated in order for production of FOS to occur in a transgenic cell, with no deleterious effect.

Expression of a gene with sucrose metabolizing activity, such as an FTF protein, in a specific transgenic plant species would not necessarily create the same, or even a desired effect when expressed in an alternate plant species. Differences in carbohydrate profiles among species suggests that an enzyme specific for sucrose will not always have sufficient substrate available to produce the same result when expressed in various species. It has been well established that the availability of sucrose as a substrate not only varies greatly from species to species but also in mutants within the same species, (Lampe et al. (1931) *Bot. Gaz*. *19*:337-380).

Mechanisms for sucrose transport and accumulation in tissue also vary greatly from one species to another. Sucrose hydrolysis is an integral part of the import mechanism in developing corn seed, (Porter et al., (1985) *Plant Phys*., *77*:524-531), but is not a prerequisite for transport to developing soybean embryo (Thorne, (1982) *Plant Phys*., *70*:953-958), or to wheat endosperm (Jenner, *Aust. J*. *Plant Phys. 1*:319-329 (1974)). Therefore, expression of a FTF in the seed of one species may have access to an abundance of sucrose, however, FOS synthesis in seed of another species could be severly hindered by the accumulation of hexose sugars in place of sucrose.

Tissue and developmental specific expression of a gene may be intrinsic to the promoter, the 3' non-coding region or combinations of the two, used in chimeric constructs. Promoters utilized to drive gene expression in transgenic plants can be derived from many sources so long as the chosen promoter(s) have sufficient transcriptional activity to accomplish the invention by expressing translatable mRNA in the desired host tissue. Preferred promoters are those that allow expression specifically in seeds. Examples of seed-specific promoters include, and are not limited to, the promoters of seed storage proteins. The seed storage proteins are strictly regulated, being expressed almost exclusively in seeds in a highly organ-specific and stage-specific manner (Higgins et al. (1984) *Ann*. *Rev. Plant Physiol*. *35*:191-221; Goldberg et al. (1989) *Cell 56*:149-160; Thompson et al. (1989) *BioEssays 10*:108-113). Moreover, different seed storage proteins may be expressed at different stages of seed development.

There are currently numerous examples for seed-specific expression of seed storage protein genes in transgenic plants. These include genes from monocots such as for barley β-hordein (Marris et al. (1988) *Plant Mol. Biol.* 10:359-366) and wheat glutenin (Colot et al. (1987) *EMBO J*. *6*:3559-3564). Moreover, promoters of seed-specific genes, operably linked to heterologous coding sequences in chimeric gene constructs, also maintain their temporal and spatial expression pattern in transgenic plants. Such examples include linking either the *Phaseolin* or *Arabidopsis* 2S albumin promoters to the Brazil nut 2S albumin coding sequence and expressing such combinations in tobacco, *Arabidopsis,* or *Brassica napus* (Altenbach et al. (1989) *Plant Mol*. *Biol*. *13*:513-522; Altenbach et al. (1992) Plant *Mol. Biol*. *18*:235-245; De Clercq et al. (1990) *Plant Physiol*. *94*:970-979), bean lectin and bean β-phaseolin promoters to express luciferase (Riggs et al. (1989) *Plant Sci. 63*:47-57), and wheat glutenin promoters to express chloramphenicol acetyl transferase (Colot et al. (1987) *EMBO J*. *6*:3559-3564).

Of particular use in the expression of the nucleic acid fragment(s) of the invention will be promoters from several extensively characterized corn seed storage protein genes such as endosperm-specific promoters from the 10 kD zein gene (Kirihara et al. (1988) *Gene 71*:359-370), the 15 kD zein gene (Hoffman et al. (1987) *EMBO J*. *6*:3213-3221; Schernthaner et al. (1988) *EMBO J*. *7*:1249-1253; Williamson et al. (1988) *Plant Physiol*. *88*:1002-1007), the 27 kD zein gene (Prat et al. (1987) *Gene 52*:51-49; Gallardo et al. (1988) *Plant Sci. 54*:211-281), and the 19 kD zein gene (Marks et al. (1985) *J*. *Biol*. *Chem.* 260:16451-16459). The relative transcriptional activities of these promoters in corn have been reported (Kodrzyck et al. (1989) *Plant Cell 1*:105-114) providing a basis for choosing a promoter for use in chimeric gene constructs for corn. Moreover, promoters that drive the expression of genes encoding enzymes involved in starch biosynthesis may be used in the practice of this invention. These include the 5' regulatory sequences of the sucrose synthase (Yang, N.-S. and Russell, D. (1990) *Proc. Natl*. *Acad*. *Sci. 87*:4144-4148) and the waxy or granule-bound starch synthase I (Unger et al. (1991) *Plant Physiol. 96*:124) genes. Promoter elements may be derived from other starch synthase (granule-bound and soluble isoforms) genes when these become available, and from the sh2 (Bhave et al. (1990) *Plant Cell 2*:581-588) and bt2 (Bae et al. (1990) *Maydica 35*:317-322) genes whose products constitute the enzyme ADP-glucose pyrophosphorylase. It is envisioned that the introduction of enhancers or enhancer-like elements into other promoter constructs will also provide increased levels of primary transcription to accomplish the invention. These would include viral enhancers such as that found in the 35S promoter (Odell et al. (1988) *Plant Mol*. *Biol*. *10*:263-272), enhancers from the opine genes (Fromm et al. (1989) *Plant Cell 1*:977-984), or enhancers from any other source that result in increased transcription when placed into a promoter operably linked to the nucleic acid fragment of the invention.

Introns isolated from the maize Adh-1 and Bz-1 genes (Callis et al. (1987) *Genes Dev. 1*:1183-1200), and intron 1 and exon 1 of the maize Shrunken-1 (sh-1) gene (Maas et al. (1991) *Plant Mol*. *Biol*. *16*:199-207) may also be of use to increase expression of introduced genes. Results with the first intron of the maize alcohol dehydrogenase (Adh-1) gene indicate that when this DNA element is placed within the transcriptional unit of a heterologous gene, mRNA levels can be increased by 6.7-fold over normal levels. Similar levels of intron enhancement have been observed using intron 3 of a maize actin gene (Luehrsen, K. R. and Walbot, V. (1991) *Mol*. *Gen. Genet. 225*:81-93). Enhancement of gene expression by Adh1 intron 6 (Oard et al. (1989) *Plant Cell Rep 8*:156-160) has also been noted. Exon 1 and intron 1 of the maize sh-1 gene have been shown to individually increase expression of reporter genes in maize suspension cultures by 10 and 100-fold, respectively. When used in combination, these elements have been shown to produce up to 1000-fold stimulation of reporter gene expression (Maas et al. (1991) *Plant Mol*. *Biol*. *16*:199-207).

Any 3' non-coding region capable of providing a polyadenylation signal and other regulatory sequences that may be required for proper expression can be used to accomplish the invention. This would include the 3' end from any storage protein such as the 3' end of the 10kd, 15kd, 27kd and alpha zein genes, the 3' end of the bean phaseolin gene, the 3' end of the soybean b-conglycinin gene, the 3' end from viral genes such as the 3' end of the 35S or the 19S cauliflower mosaic virus transcripts, the 3' end from the opine synthesis genes, the 3' ends of ribulose 1,5-bisphosphate carboxylase or chlorophyll a/b binding protein, or 3' end sequences from any source such that the sequence employed provides the necessary regulatory information within its nucleic acid sequence to result in the proper expression of the promoter/coding region combination to which it is operably linked. There are numerous examples in the art that teach the usefulness of different 3' non-coding regions (for example, see Ingelbrecht et al. (1989) *Plant Cell 1*:671-680).

Numerous genes from microbial (Fouet, A., Arnaud, M., Klier, A. and Rapoport, G., (1984) *Biochem. Biophys. Res. Commun. 119*, 795-800; Shiroza, T. and Kuramitsu, H. K., (1988) J. *Bacteriol. 170*, 810-816; Tang, L. B., Lenstra, R., Borchert, T. V. and Nagarajan, V. (1990) *Gene 96,* 89-93) and plant sources (Vijn et al., (1997) *Plant J*. *11*:387-398; Sprenger et al., (1997) *Febs Lett. 400*:355-358; Van Tunen et al., WO 96/21023; Smeekens et al., WO 96/01904), encoding enzymes with FIT activity have been isolated and sequenced. Preferred among these are the microbial-derived FTF genes from *Bacillus amyloliquefaciens*.

The FTF genes can be isolated by techniques routinely employed by the skilled artisan for isolation of genes when the nucleotide sequence of the desired gene is known, or when the sequence of a homologous gene from another organism is known. Sequence information about the desired gene can be used to prepare oligonucleotide probes for identification and isolation of the entire gene from an appropriate genetic library. This library may be a genomic library, wherein the coding region may be contained on a single DNA fragment or may be contained on several distinct DNA fragments. Alternatively, the library may be a cDNA library wherein the likelihood of isolating a cDNA clone comprising the entire coding region as one contiguous sequence is greater. In either instance, the appropriate clone(s) can be identified by DNA-DNA hybridization with probes corresponding to one or more portions of the desired genes. Alternatively, oligonucleotide primers can be prepared and employed as PCR primers in order to amplify and subsequently isolate all or part of the coding region from genomic DNA, or from the genomic or cDNA libraries described above.

Several different assays can be used to detect expression of the chimeric genes in seeds of the transformed plants. RNA transcripts, specific to the FTF genes may be detected by Southern or northern analysis. The FTF protein can be extracted, detected and quantified immunologically by methods known to those skilled in the art. Alternatively seed tissue may be ground and extracted with a polar solution, isolating and concentrating polysaccharides, including FOSs, which can then be detected by: TLC analysis, combined with a kestose specific stain (Wise et al., (1955) *Anal. Chem. 27*:33-36); HPLC analysis using FOS standards (Chatterton et al. (1993) In: Fuchs A. ed. *Inulin and inulin-containing crops.* Elsevier, Amsterdam pp. 93-99); or hydrolysis followed and an enzymatic-linked assay (Brown, C. and Huber, S. (1987) *Physiol*. *Plant 70*:537-543).

Various methods of introducing a DNA sequence (chimeric constructs containing FTF genes) into eukaryotic cells (i.e., transformation) of higher plants are available to those skilled in the art (see EPO publications 0 295 959 A2 and 0 138 341 A1). Such methods include high-velocity ballistic bombardment with metal particles coated with the nucleic acid constructs (see Klein et al. (1987) *Nature* (London) *327*:70-73, and see U.S. Patent No. 4,945,050), as well as those based on transformation vectors based on the Ti and Ri plasmids of *Agrobacterium spp.*, particularly the binary type of these vectors. Ti-derived vectors transform a wide variety of higher plants, including monocotyledonous and dicotyledonous plants, such as soybean, cotton and rape (Pacciotti et al. (1985) *Bio*/*Technology 3*:241; Byrne et al. (1987) *Plant Cell, Tissue and Organ Culture 8*:3; Sukhapinda et al. (1987) *Plant Mol*. *Biol*. *8*:209-216; Lorz et al. (1985) *Mol. Gen. Genet. 199*:178-182; Potrykus et al. (1985) *Mol. Gen. Genet. 199*:183-188).

Other transformation methods for chimeric constructs containing FTF genes are available to those skilled in the art, such as direct uptake of foreign DNA constructs (see EPO publication 0 295 959 A2), and techniques of electroporation (see Fromm et al. (1986) *Nature* (London) *319*:791-793). Once transformed, the cells can be regenerated by those skilled in the art. Also relevant are several recently described methods of introducing nucleic acid fragments into commercially important crops, such as rapeseed (see De Block et al. (1989) *Plant Physiol*. *91*:694-701), sunflower (Everett et al., (1987) *Bio*/*Technology 5*:1201-1204), soybean (McCabe et al. (1988) *Bio*/*Technology 6*:923-926; Hinchee et al. (1988) *Bio*/*Technology 6*:915-922; Chee et al. (1989) *Plant Physiol*. *91*:1212-1218; Christou et al. (1989) *Proc. Natl. Acad. Sci USA 86*:7500-7504; EPO Publication 0 301 749 A2), and corn (Gordon-Kamm et al. (1990) *Plant Cell 2*:603-618; Fromm et al. (1990) *Bio*/*Technology 8*:833-839). One skilled in the art is familiar with still other means for the production of transgenic maize plants including introduction of DNA into protoplasts and regeneration of plants from said protoplasts (Omirulleh et al. (1993) *Plant Mol*. *Biol*. *21*:415-423), electroporation of intact tissues (D'Hulluin et al. (1992) *Plant Cell 4*:1495-1505; Laursen et al. (1994) *Plant Mol. Biol*. *24*:51-61), silica carbide mediated fiber transformation of maize cells (Kaeppler et al. (1992) *Theor. Appl. Genet. 84*:560-566; Frame et al. (1994) *Plant J*. *6*:941-948). In addition to the method of particle bombardment of maize callus cells described above, one skilled in the art is familiar with particle bombardment of maize scutellar or suspension cultures to yield fertile transgenic plants (Koziel et al. (1993) *Bio*/*Technology 11*:194-200; Walters et al. (1992) *Plant Mol*. *Biol*. *18*:189-200).

Once transgenic plants are obtained by one of the methods described above, it will be necessary to screen individual transgenics for those that most effectively display the desired phenotype. It is well known to those skilled in the art that individual transgenic plants carrying the same construct may differ in expression levels; this phenomenon is commonly referred to as "position effect". For example, when the construct in question is designed to express higher levels of the gene of interest, individual plants will vary in the amount of the protein produced and thus in enzyme activity; this in turn will effect the phenotype. This should not be seen as a limitation on the present invention, but instead as practical matter that is appreciated and anticipated by the person skilled in this art. Accordingly, skilled artisans will develop methods for screening large numbers of transformants. The nature of these screens will generally be chosen on practical grounds, and is not an inherent part of the invention.

### EXAMPLES

The present invention is further defined in the following examples. It will be understood that the examples are given for illustration only and the present invention is not limited to uses described in the examples. The present invention can be used to generate transgenic corn plants whose seed carbohydrate profile is altered by accumulation of fructose polymers and where its properties are useful such as in, but not limited to, foods, paper, plastics, adhesives, or paint.

### EXAMPLE 1

### Chimeric Construct for Expression of the Plastid-targeted Bacillus amyloliquefaciens FTF Gene in transgenic Zea mays L.

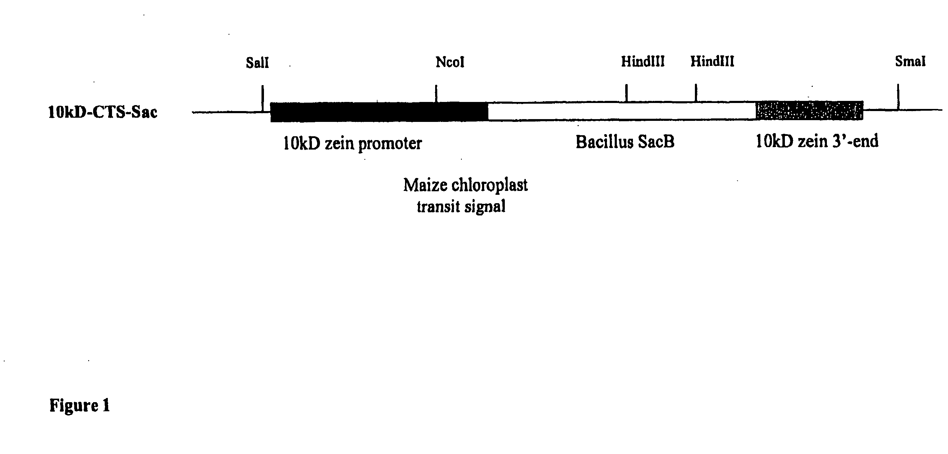

Construction of the corn endosperm, specific expression vector utilized in this specification was accomplished by isolating a region of maize DNA encoding a RuBP-carboxylase chloroplast-targeting signal (described in U.S. Patent No. 5,773,691). The chloroplast-targeting signal consists of a 47 amino acid peptide fragment (SEQ ID NO:2) encoded by a portion of the maize RuBP-carboxylase (RUBISCO) gene. A 183 bp DNA fragment (SEQ ID NO:1) encoding the chloroplast-targeting signal was isolated and subsequently ligated into the expression vector pCyt-Sac (Caimi et al., *Plant Physiology 110*:355-363 (1996), previously digested with the enzymes NcoI and EcoRV. The final construct (10kD-CTS-Sac) contained an endosperm specific 10 kD zein promoter and 3' end, in addition to the chloplast-targeting signal, fused in frame, to the *Bacillus* *amyloliquefaciens* SacB gene (Figure 1), which encodes the FTF gene. A SacB gene useful in this invention has been described in Tang et al., (1990) *Gene 96*:89-93; Nagarajan et al., U.S. Patent No. 5,162,2070 and Caimi et al., WO 95 13,389). The source of DNA from which the FTF gene was isolated from *Bacillus amyloliquefaciens* available from the American Type Culture Collection, (Manassas, VA)

The maize endosperm expression cassette (10kD-CTS-Sac), containing the 10 kD zein promoter, chloroplast targeting signal, SacB coding sequence and 10 kD 3' end, was isolated by digesting with SmaI and BamHI, then ligated into a plasmid vector in preparation for transformation into maize.

### Plant material and transformation

The chimeric gene described above was then introduced into corn cells by the following procedure. Immature corn embryos were dissected from developing caryopses derived from crosses of the inbred corn lines H99 and LH132 (Gerdes et al., (1993) Compilation of North American Maize Breeding Germplasm. Editorial committee, W.F. Tracy, et al., Crop Science Society of America, Inc. Madison, WI). The embryos were isolated 10 to 11 days after pollination when they were 1.0 to 1.5 mm long. The embryos were then placed with the axis-side facing down and in contact with agarose-solidified N6 medium (Chu et al., (1975) *Sci. Sin. Peking 18*:659-668). The embryos were kept in the dark at 27°C and were characterized as friable embryogenic callus consisting of undifferentiated masses of cells with somatic proembryoids and embryoids borne on suspensor structures proliferating from the scutellum of the immature embryos. The embryogenic callus isolated from the primary explant was cultured on N6 medium and sub-cultured on this medium every 2 to 3 weeks.

A plasmid, p35S/Ac (obtained from Dr. Peter Eckes, Hoechst Ag, Frankfurt, Germany), harboring the *pat* gene (which encodes phosphinothricin acetyl transferase, see European Patent Publication 0 242 236) was used in transformation experiments in order to provide for a selectable marker. The enzyme PAT confers resistance to herbicidal glutamine synthetase inhibitors such as phosphinothricin. The *pat* gene was placed under the control of the 35S promoter from Cauliflower Mosaic Virus (Odell et al. (1985) *Nature 313*:810-812) and the 3' region of the nopaline synthase gene from the T-DNA of the Ti plasmid of Agrobacterium tumefaciens.

The particle bombardment method (Klein et al., (1987) *Nature 327*:70-73) was used to transfer genes to the callus culture cells. According to this method, gold particles (1 µm in diameter) were coated with DNA using the following technique. Ten µg of plasmid DNA was added to 50 µL of a suspension of gold particles (60 mg per mL). Calcium chloride (50 µL of a 2.5 M solution) and spermidine free base (20 µL of a 1.0 M solution) were added to the particles. The suspension was vortexed during the addition of these solutions. After 10 minutes, the tubes were briefly centrifuged (5 sec at 15,000 rpm) and the supernatant removed. The particles were suspended in 200 µL of absolute ethanol, centrifuged again and the supernatant removed. The ethanol rinse was performed again and the particles suspended in a final volume of 30 µL of ethanol. An aliquot (5 µL) of the DNA-coated gold particles was placed in the center of a Kapton™ flying disc (Bio-RDA Labs). The particles were then accelerated into the corn tissue with a Biolistic™ PDS-1000/He (Bio-Rad Instruments, Hercules CA), using a helium pressure of 1000 psi, a gap distance of 0.5 cm and a flying distance of 1.0 cm.

For bombardment, the embryogenic tissue was placed on filter paper over agarose-solidified N6 medium. The tissue was arranged as a thin lawn and covered a circular area of about 5 cm in diameter. The petri dish containing the tissue was placed in the chamber of the Biolistic™ PDS-1000/He approximately 8 cm from the stopping screen. The air in the chamber was then evacuated to a vacuum of 28 inches of Hg. The macrocarrier was accelerated with a helium shock wave using a rupture membrane that bursts when the He pressure in the shock tube reaches 1000 psi.

Seven days after bombardment the tissue was transferred to N6 medium that contains glufosinate (2 mg per liter) and lacks casein or proline. The tissue continued to grow slowly on this medium. After an additional 2 weeks the tissue was transferred to fresh N6 medium containing gluphosinate. After 6 weeks, areas of about 1 cm in diameter of actively growing callus was identified on plates containing the glufosinate-supplemented medium. Calli continued to grow when sub-cultured on the selective medium.

Plants were regenerated from the transgenic callus by first transferring clusters of tissue to N6 medium supplemented with 0.2 mg per liter of 2,4-D. After two weeks the tissue was transferred to regeneration medium (Fromm et al., (1990) *Bio*/*Technology 8*:833-839).

### Analysis of transgenic maize Lines Containing the 10kD-CTS-Sac chimeric gene

Characterization and confirmation of FTF activity within transgenic tissue has been described in Caimi et al., WO 95 13389; Caimi et al., (1996) *Plant Physiol*. *110*:355-363; Caimi et al., (1997) *New Phytol. 136*:19-28.

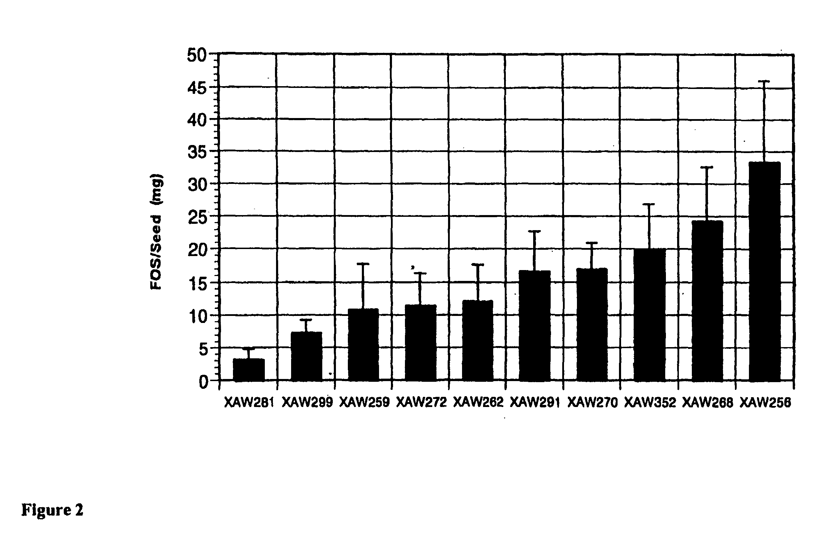

Individual mature seeds were analyzed for the presence and concentration of FOS by grinding to a fine powder and suspending in 80% ethanol in water. The solution was heated to 65°C for 10 minutes and centrifuged at 2500 x g. The supernatant, containing soluble carbohydrates, was discarded and the pellet suspended in 80% ethanol. The soluble carbohydrate extraction step was repeated twice. The resulting pellet was extracted with water and the FOS content determined by the resorcinol method described in Yaphe and Arsenault (1965) *Anal Biochem 13*:143-148. Examples of FOS concentration in seeds containing the 10kD-CTS-Sac expression cassette are shown in Figure 2.

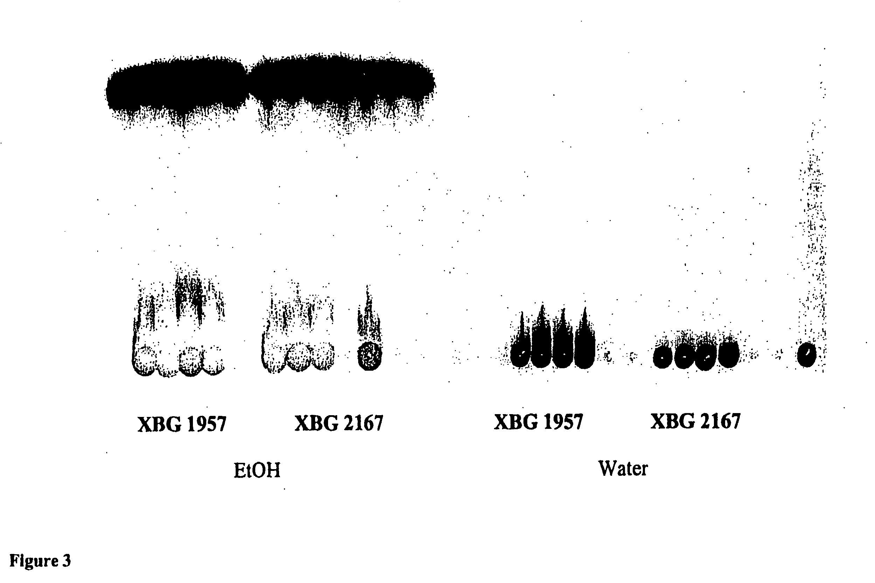

Additional characterization of FOS from single seeds was accomplished by spotting 3-5 ul of the water extraction, described above, on a TLC plate. The TLC plate was developed twice by the method of Wise et al. ((1955) *Anal Chem 27*:33-36) in butanol:propanol:water (12:3:4). FOS signals were visualized after spraying the plate with a urea/phosphoric acid mixture (Wise et al. (1955) *Anal Chem 27*:33-36) and heating to 100°C for ten minutes. Positive signals at the plate origin demonstrate the presence of high molecular weight FOS (Figure 3).

### EXAMPLE 2

### Starch Analysis of Transgenic Lines containing the 10kD-CTS-Sac Chimeric Gene

Because endosperm plastids of corn normally accumulate large amounts of starch, a polymer of glucose, it was of interest to determine if the starch in FOS accumulating lines was altered in structure or functionality. Starch was extracted from single seeds obtained from corn plants transformed with the 10kD-CTS-Sac construct. Seeds were steeped in a solution containing 1.0% lactic acid and 0.3% sodium metabisulfite, pH 3.82, and held at 52°C for 22-24 h. Seeds were drained, rinsed and homogenized individually in 8-9 mL of a 100 mM NaCl solution. Five mL of toluene was added to each tube, the tubes were vigorously shaken twice for 6 minutes. The solutions were then allowed to settle for 30 minutes. Two mL of 100 mM NaCl was added to each tube and the solutions were allowed to settle for another 30 minutes. The protein-toluene layer was next aspirated off. The toluene wash step was repeated. Twelve mL of water was added to each tube and the mixtures shaken in a paint shaker for 45 seconds. The solutions were centrifuged for 10 minutes in a table top centrifuge and then the water was removed. The water wash was repeated, followed by a final wash with 12 mL of acetone. After shaking and centrifugation steps, the acetone was drained and allowed to evaporate for 1 h. Starch extracts were incubated in a 40°C oven overnight to drive off any remaining acetone.

### Microscopic Observation of Isolated Starch Granules

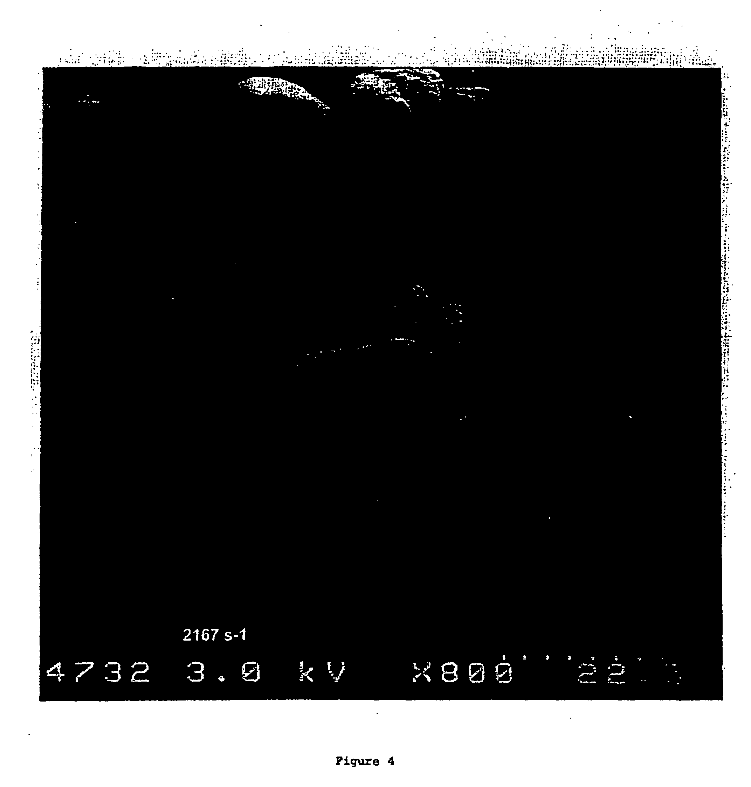

Scanning Electron Microscopy was used to examine the morphology of intact starch granules isolated from FOS accumulating and non-accumulating seeds. Figure 4 shows micrographs of starch granules from two representative lines. XBG02167-1 is a seed, which lacks FOS. Starch granules from this line appear normal, having the polygonal appearance typical of cornstarch granules. XBG02167-2 is a FOS accumulating seed. It can be seen that starch granules from this line have a very different appearance. The granules are elongated and irregular in shape. They are also generally smaller than the granules from seed XBG02167-1. The surface of the granules is also highly irregular and much rougher in appearance the granules from normal dent corn.

### Structural Analysis of Starch

Extracted starches were enzymatically debranched as follows. Seven mg of each starch sample was added to a screw cap test tube with 1.1 mL of water. The tubes were heated to 120°C for 30 minutes and then placed in a water bath at 45°C. Debranching solution was made by diluting 50 µL of isoamlyase (5x10⁶ units/mL, Sigma) per mL of sodium acetate buffer (50 mM, pH 4.5). 40 µL of debranching solution was added to each starch sample and incubated for 3 h at 45°C. Reactions were terminated by heating to 110°C for 5 minutes. Debranched starch samples were lyophilized and dissolved in DMSO for analysis by gel permeation chromatography (GPC). Ten µL of debranched starch was injected and run through 3 narrow-bore columns (Polymer Labs, Mini-Mix C, D, E with a Mini-mix C guard column) in series at 90°C and eluted with DMSO at a flow rate of 0.35 mL/min. Sampling interval was 35 minutes. A refractive index detector (Waters) was used with a computer running Waters Millenium Chromatography Manager System with GPC option (version 2.15.1, Waters Corp.) for detection and data collection and analysis, respectively. Retention times of pullulan standards (Standard 1: 380K, 100K, 23.7K, 5.8K, 666 and 180 mw, Standard 2: 853K, 186K, 48K, and 12.2K) were used to establish a 3^{rd} order calibration and calculate molecular weight distributions within the Millenium Software.

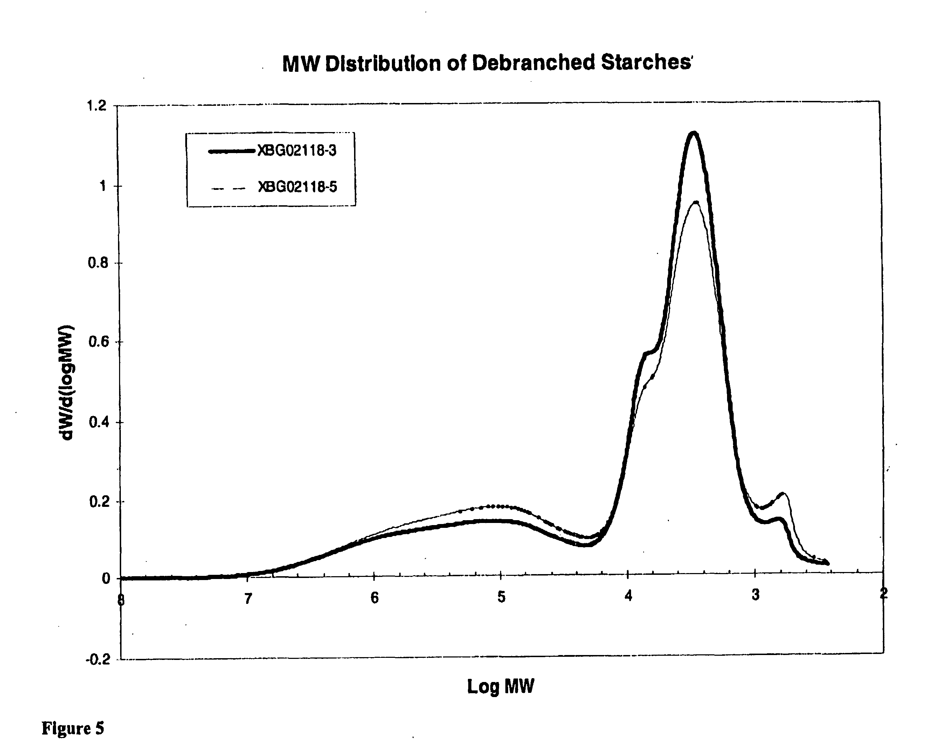

As is known to those skilled in the art transgenic corn plants produced by particle bombardment are typically hemizygous for the introduced transgene and will segregate the transgene in a predictable Mendelian fashion. On the selfed ear of a R0 plant the triploid endosperm, which is the tissue responsible for starch production, will segregate the introduced transgene. Three segregants that contained FOS (XBG02118-5, XBG02118-6, and XBG02118-7) and 4 seeds that did not contain FOS (XBG02118-1, XBG02118-2, XBG02118-3, and XBG02118-4) were selected from a selfed ear of R0 plant XBG02118 were extracted for starch and analyzed for starch structure as described above. Figure 5 shows the molecular weight distributions of representative debranched starches from FOS accumulating and non-accumulating seeds.

### Molecular weight distributions of representative debranched starches from FOS accumulating and non-accumulating seeds.

As can be seen in Figure 5 line XBG02118 produces starches with two different types of Molecular Weight Distributions. The molecular weight distributions of debranched starch from seeds of both FOS accumulating and non-accumulating lines are generally typical of the molecular weight distribution observed for normal dent corn starch. However, the FOS accumulating seeds (i.e. XBG02118-5) contain a slight increase in the proportion of material of log MW > 4.2 relative to the amount of material of log MW < 4.2. The material of log MW > 4.2 is classified as amylose and the material of log MW < 4.2 is classified as amylose (based on comparison of starches from normal corn and waxy corn). Because RI detection is non-selective the excess high MW material could be amylose (alpha 1-4, linked glucose polymer), FOS polymer, or aberrant starch that is partially resistant to the isoamylase. The proportion of material of log MW > 4.2 to material of log MW < 4.2 was compared for three different FOS accumulating lines by debranching analysis and integration using the Millenium software.

**TABLE 1**

| Quantitative Comparison of High MW Material component of Debranched Starches from seeds with and without FOS | | | |
|---|---|---|---|
| | Mass % of Material | | |
| Line | | Log MW >4.2 | Std Error |
| XBG02045 | Fructan | 30.16 | 0.47 |
| | No Fructan | 24.03 | 0.30 |
| | | | |
| XBG02167 | Fructan | 38.63 | 1.60 |
| | No Fructan | 24.85 | 0.31 |
| | | | |
| XBG02118 | Fructan | 31.35 | 0.48 |
| | No Fructan | 24.38 | 0.22 |

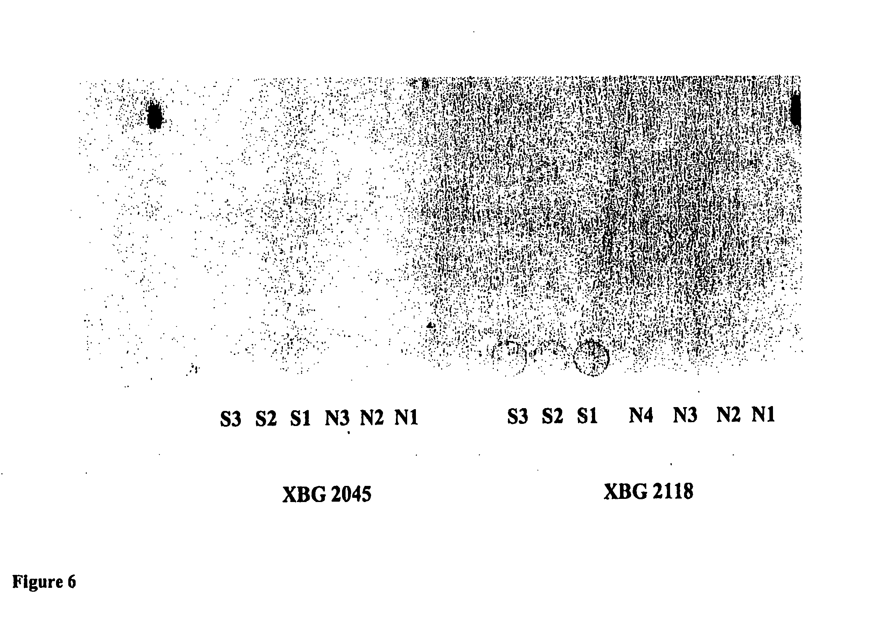

This result demonstrates that plastid targeting of the SacB gene product in corn endosperm not only results in accumulation of FOS but also gives rise to an alteration in the structure of starch produced by these seeds. To determine whether FOS polymer could be recovered from the purified starch the samples were suspended in water and heated to 65°C for 10 minutes. The slurry was centrifuged at 5000 x g for 10 minutes and 3 ul of supernatant spotted on a TLC plate. FOS visualization was accomplished by the methods described in Example 1 above. The TLC plate (Figure 6) demonstrates the presence of FOS in the processed starch samples.

### EXAMPLE 3

### Functional Analysis of Starch Produced by seeds expressing plastid targeted SacB.

In order to obtain a larger amount of starch for functional analysis 15 g of FOS containing kernels were identified from two lines carrying the plastid targeted SacB (XAW00361, XAW00363). For each line 15 g of kernels were weighed into a 50 mL Erlenmeyer flask and steeped in 50 mL of steep solution (see Example 2) for 18 h at 52°C. The kernels were then drained and rinsed with water. The kernels were homogenized using a 20 mm Polytron probe (Kinematica GmbH; Kriens-Luzern, Switzerland) in 50 mL of cold 50 mM NaCl. The homogenate was filtered through a 72 micron mesh screen. The filtrate was brought up to a total volume of 400 mL with 50 mM NaCl and an equal volume of toluene was added. The mixture was then stirred with a magnetic stir bar for 1 h at sufficient speed to completely emulsify the two phases. The emulsion was allowed to separate overnight in a covered beaker. The upper toluene layer was aspirated from the beaker and discarded. The starch slurry remaining in the bottom of the beaker was suspended, poured into a 250 mL centrifuge bottle and centrifuged 15 minutes at 25,000 RCF. The supernatant was discarded and the starch was washed sequentially with water and acetone by shaking and centrifuging as above. After the acetone wash and centrifugation the acetone was decanted and the starch allowed to dry overnight in a fume hood at room temperature.

A Rapid Visco Analyzer 4 (RVA) (Newport Scientific; Sydney, Australia) and Thermocline software for Windows (Version 2.0) was used for pasting curve analysis and the starches from FOS accumulating lines were compared to starch from normal dent corn. For each line, 1.5 ± .0025 g of starch was placed into a new aluminum RVA sample can and 25 ± .1 mL of .66% sodium phosphate (dibasic, heptahydrate) buffer, pH 6.5 was added. The moisture content of the starch was assumed to be 10%, giving a 5% total solids concentration. A stir lid was placed onto the can and the slurry agitated by spinning the lid. The sample is then run under the following profile:

| | | |
|---|---|---|
| Paddle Speed | 0-10s | 960RPM |
| | 11s-16min | 160RPM |
| | | |
| Temperature | 0-1min. | 50°C |
| | 1min.-5min. | 50°C→95°C |
| | 5min.-8min. | 95°C |
| | 8min.-12min. | 95°C→50°C |
| | 12min.-16min. | 50°C |

(The single temperature indicates holding at that target temperature. The dual temperature listing indicates start and end temperatures over the selected time frame with a constant heating rate increase.)

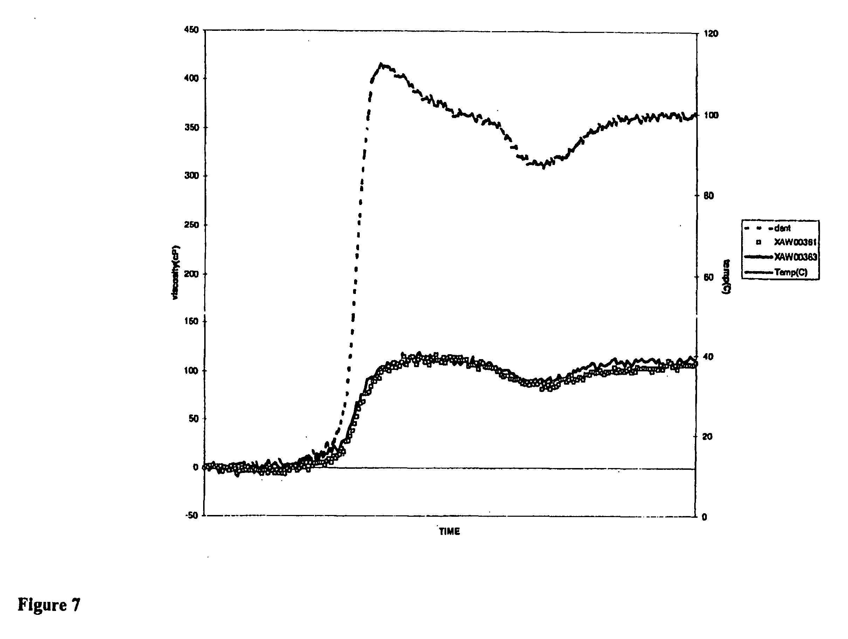

Figure 7 shows the pasting curves of normal dent corn starch and the starch from FOS containing lines. The starches from the FOS accumulating lines have very different pasting properties than starch from control corn. The onset of pasting is at a higher temperature than for control corn and the peak viscosity is lower. Thus, targeting the SacB gene product to endosperm plastids not only causes accumulation of high levels of FOS but also alters the functionality of starch produced in the same plastids.

## Claims

1. A recombinant DNA construct comprising a tissue specific promoter, operably linked to a plastid targeting sequence, operably linked to a coding sequence for a fructosyltransferase gene such that said construct can transform a monocot cell to obtain the production of fructo-oligosaccharides in the plastid of said monocot cell.

2. A recombinant DNA construct of Claim 1 wherein said monocot is selected from the group consisting of corn, wheat, rice, barley, sorghum, triticale and rye.

3. A monocot transformed with a construct of Claim 1 such that said monocot produces fructo-oligosaccharides which accumulate in the plastid within cells of said monocot.

4. A method of producing fructo-oligosaccharides comprising growing the monocot of Claim 3, harvesting said monocot, and extracting said fructo-oligosaccharides from the harvested monocot.

5. A recombinant DNA construct as described in Claim 1 wherein the tissue specific promoter is specific to seed.

6. A monocot transformed with the construct of Claim 1 wherein said monocot produces fructo-oligosaccharides which accumulate in a seed of said monocot.

7. A method of increasing fructo-oligosaccharide levels in a monocot, comprising inserting into a monocot cell a recombinant DNA construct comprising a tissue specific promoter, operably linked to a plastid targeting sequence, operably linked to a coding sequence for a fructosyltransferase gene, such that the construct can transform a monocot cell and the transformed monocot synthesizes and accumulates fructo-oligosaccharides.

8. A method of Claim 7 wherein said monocot is selected from the group consisting of corn, wheat, rice, barley, sorghum, triticale and rye.

9. A monocot transformed with the construct of Claim 1.

10. A seed produced from the transformed monocot of Claim 7.

11. A method of making a starch fructo-oligosaccharide blend comprising inserting into a monocot cell a recombinant DNA construct comprising a tissue specific promoter, operably linked to a plastid targeting sequence, operably linked to a coding sequence for a fructosyltransferase gene to produce a transgenic monocot plant, growing said plant, harvesting said plant, and extracting the fructo-oligosaccharide/starch blend from said harvested plant.

## Patentansprüche

1. Rekombinantes DNA-Konstrukt, umfassend einen gewebespezifischen Promotor, der funktionsfähig mit einer Plastid-Targeting-Sequenz verknüpft ist, die funktionsfähig mit einer Kodiersequenz für ein Fructosyltransferase-Gen dergestalt verknüpft ist, dass das Konstrukt zum Erhalt der Produktion von Fructooligosacchariden im Plastid der Monocotyledon-Zelle eine Monocotyledon-Zelle transformieren kann.

2. Rekombinantes DNA-Konstrukt nach Anspruch 1, worin der Monocotyledon aus der Gruppe ausgewählt ist, bestehend aus Mais, Weizen, Reis, Gerste, Sorghum, Tritical und Roggen.

3. Mit einem Konstrukt nach Anspruch 1 dergestalt transformierten Monocotyledon, dass der Monocotyledon Fructooligosaccharide produziert, die sich im Plastid in Zellen des Monocotyledons akkumulieren.

4. Verfahren zum Produzieren von Fructooligosacchariden umfassend das Anbauen des Monocotyledons nach Anspruch 3, Ernten des Monocotyledons und Extrahieren der Fructooligosaccharide aus dem geernteten Monocotyledon.

5. Rekombinantes DNA-Konstrukt nach Anspruch 1, worin der gewebespezifische Promotor für Samen spezifisch ist.

6. Mit dem Konstrukt nach Anspruch 1 transformierten Monocotyledon, worin der Monocotyledon Fructooligosaccharide produziert, die sich in einem Samen des Monocotyledons akkumulieren.

7. Verfahren zur Erhöhung der Fructooligosaccharid-Konzentrationen in einem Monocotyledon, umfassend das Insertieren eines rekombinanten DNA-Konstrukts in eine Monocotyledon-Zelle, umfassend einen gewebespezifischen Promotor, der funktionsfähig mit einer Plastid-Targeting-Sequenz verknüpft ist, die funktionsfähig mit einer Kodiersequenz für ein Fructosyltransferase-Gen dergestalt verknüpft ist, dass das Konstrukt eine Monocotyledon-Zelle transformieren kann und der transformierte Monocotyledon Fructooligosaccharide synthetisiert und akkumuliert.

8. Verfahren nach Anspruch 7, worin der Monocotyledon aus der Gruppe ausgewählt ist, bestehend aus Mais, Weizen, Reis, Gerste, Sorghum, Tritical und Roggen.

9. Ein mit dem Konstrukt nach Anspruch 1 transformierten Monocotyledon.

10. Aus dem transformierten Monocotyledon nach Anspruch 7 produzierter Samen.

11. Verfahren zur Herstellung einer Stärke-Fructooligosaccharid-Mischung, umfassend das Insertieren eines rekombinanten DNA-Konstrukts in eine Monocotyledon-Zelle, umfassend einen gewebespezifischen Promotor, der funktionsfähig mit einer Plastid-Targeting-Sequenz verknüpft ist, die funktionsfähig mit einer Kodiersequenz für ein Fructosyltransferase-Gen zur Produktion einer transgenen monocotyledonen Pflanze verknüpft ist, Anbauen der Pflanze, Ernten der Pflanze und Extrahieren der Fructooligosaccharid/Stärke-Mischung aus der geernteten Pflanze.

## Revendications

1. Produit de synthèse d'ADN recombinant comprenant un promoteur tissulaire spécifique, lié de manière opérationnelle à une séquence ciblant un plaste, lié de manière opérationnelle à une séquence codant un gène de fructosyl-transférase de sorte que ledit produit de synthèse peut transformer une cellule monocotylédone pour obtenir la production de fructo-oligosaccharides dans le plaste de ladite cellule monocotylédone.

2. Produit de synthèse d'ADN recombinant de la revendication 1, dans lequel ladite monocotylédone est sélectionnée parmi le groupe consistant en maïs, blé, riz, orge, sorgho, triticale et seigle.

3. Monocotylédone transformée avec un produit de synthèse de la revendication 1, de sorte que ladite monocotylédone produit des fructo-oligosaccharides qui s'accumulent dans le plaste à l'intérieur des cellules de ladite monocotylédone.

4. Méthode de production de fructo-oligosaccharides comprenant cultiver la monocotylédone de la revendication 3, récolter ladite monocotylédone et extraire lesdits fructo-oligosaccharides de la monocotylédone récoltée.

5. Produit de synthèse d'ADN recombinant tel que décrit dans la revendication 1, dans lequel le promoteur tissulaire spécifique est spécifique à la graine.

6. Monocotylédone transformée avec le produit de synthèse de la revendication 1, où ladite monocotylédone produit des fructo-oligosaccharides qui s'accumulent dans la graine de ladite monocotylédone.

7. Méthode d'accroissement des taux de fructo-oligosaccharides dans une monocotylédone, comprenant introduire dans une cellule monocotylédone, un produit de synthèse d'ADN recombinant comprenant un promoteur tissulaire spécifique, lié de manière opérationnelle à une séquence ciblant un plaste, lié de manière opérationnelle à une séquence codant un gène de fructosyl-transférase, de sorte que le produit de synthèse peut transformer une cellule monocotylédone et que la monocotylédone transformée synthétise et accumule des fructo-oligosaccharides.

8. Méthode de la revendication 7, dans laquelle ladite monocotylédone est sélectionnée parmi le groupe consistant en maïs, blé, riz, orge, sorgho, triticale et seigle.

9. Monocotylédone transformée avec le produit de synthèse de la revendication 1.

10. Graine produite de la monocotylédone transformée de la revendication 7.

11. Méthode de fabrication d'un mélange d'amidon fructo-oligosaccharides comprenant introduire dans une cellule monocotylédone un produit de synthèse d'ADN recombinant comprenant un promoteur tissulaire spécifique, lié de manière opérationnelle à une séquence ciblant un plaste, lié de manière opérationnelle à une séquence codant un gène de fructosyl-transférase pour produire une plante monocotylédone transgénique, cultiver ladite plante, récolter ladite plante et extraire le mélange de fructo-oligosaccharides/amidon de ladite plante récoltée.


### SEQUENCE LISTING

<110> E.I. du Pont de Nemours and Company
<120> Fructose Polymer Synthesis in Monocot Plastids
<130> BB1347
<140>
   <141>
<150> 60/166,268
   <151> 1999-11-18
<160> 2
<170> Microsoft Office 97
<210> 1
   <211> 183
   <212> DNA
   <213> Zea mays
<400> 1
   atggcgccca ccgtgatgat ggcctcgtcg gccaccgccg tcgctccgtt ccaggggctt  60
   aagtccaccg ccagcctccc cgtcgcccgc cgctcctcta gaagcctcgg caacgtcagc 120
   aacggcggaa gaatccggtg catggccagt gtgattgcgc aggcaggggc gaaaggtcgt 180
   cag                                                               183
<210> .2
   <211> 47
   <212> PRT
   <213> Zea mays
<400> 2
   Met ala Pro Thr Val Met Met Ala Ser Ser Ala Thr Ala Val Ala Pro
    1               5                  10                  15
   Phe Trp Gly Leu Lys Ser Thr Ala Ser Leu Pro Val Ala Arg Arg Ser
               20                  25                  30
   Ser Arg Ser Leu Gly Asn Val Ser Asn Gly Gly Arg Ile Arg Cys
           35                  40                  45